# EUROPÄISCHE PATENTSCHRIFT

(11) **EP 0 353 336 B1**
(45) Veröffentlichungstag und Bekanntmachung des Hinweises auf die Patenterteilung: **03.11.1993**
(21) Anmeldenummer: 88112909.2
(22) Anmeldetag: 09.08.1988
(51) Int. Cl.: A61F 2/64

(54) **Künstliches Kniegelenk**
Artificial knee joint
Articulation artificielle de genou

(30) Priorität: 03.08.1988 DE 8809910 U
(43) Veröffentlichungstag der Anmeldung: 07.02.1990
(73) Patentinhaber: IPOS GMBH & CO. KG., D-21337 Lüneburg (DE)
(72) Erfinder: Prahl, Gregor Mark, D 2172 Rullstorf (DE); Schneider, Reinhold, D 2123 Bardowick (DE)
(74) Vertreter: Richter, Werdermann & Gerbaulet

(56) Entgegenhaltungen:
- FR-A- 2 187 279
- US-A- 3 934 273
- US-A- 4 135 254
- US-A- 4 549 318

## Beschreibung

Die Erfindung betrifft ein Künstliches Kniegelenk mit einem Klemmkörper, der gegen einen in einem Kniegelenkgehäuse (14) befindlichen Bremskörper anlegbar ist, und einem Mittel zur Aufnahme des Unterschenkelteils einer Beinprothese aufweisenden Teil sowie einem das Kniegelenk in die Streckposition rücktreibenden Streckzug, wobei das Kniegelenkgehäuse im hinteren Bereich um einen im wesentlichen zylindrischen, eine horizontale Achse aufweisenden Walzenkörper drehbar gelagert ist, dessen Stirnseiten (20) drehgesichert mit Teilen des Teils zur Aufnahme des Unterschenkelteils fest verbunden sind, und im vorderen Bereich eine Schwenkachse aufweist, um die ein Oberschenkelteil der Prothese derart zwischen einer Beuge- und einer Steckposition schwenkbar gelagert ist, daß der Klemmkörper (13) bei vertikaler Belastung gegen den Bremskörper zur eine weitere Flexion (Beugebewegung) verhindernden sperrenden Anlage kommt.
Ein solches Kniegelenk ist aus der US-A-4135254 bekannt.

Grundsätzliche Zielsetzung bei einer Prothese ist es, daß diese den natürlichen Bewegungsablauf des ersetzten Körpergliedes möglichst weitgehend angenähert bewirkt. Bei Beinprothesen stellt sich hierbei insbesondere das Problem, daß die nach dem Stand der Technik bekannten Belastungsbremssysteme dem Sicherheitsbedürfnis alter Menschen nicht optimal gerecht werden können. Allzu leicht werden durch Reibungsschlußprozesse dynamische Fortbewegungsprozesse, wie sie beim natürlichen Gangablauf entstehen, unphysiologisch gestört mit der Folge, daß diese Belastungsbremssysteme zu Stürzen der Prothesenträger führen.

Darüber hinaus hat bei den nach dem Stand der Technik be.-kannten Kniegelenkkontruktionen die Verkleinerung der Gelenke inzwischen zu Dimensionen geführt, bei denen Bremsleistungen, die ein gebeugtes Knie halten können, zu einer Überbeanspruchung der Gelenkkonstruktion und des Materials führen. Dies hat nicht selten einen hohen Verschleiß der Reibungsflächen, geräuschvoll arbeitende Kniegelenke und eine erhöhte Wartung zur Folge.

In einer Kniestruktur für eine Beinprothese nach der US-A-3,934,273, die es gestattet, daß das Unterschenkelteil in Bezug auf das Oberschenkelteil mittels einer Achse angewinkelt werden kann, die in dem Unterschenkelteil befestigt ist und durch entsprechend angebrachte Öffnungen in dem Oberschenkelteil geführt ist, wird ein Bremsband in einem Umkreis von fast 360° um die Achse gelegt, wobei das untere Bandende drehbar um einen in dem Oberschenkelteil parallel zu der Achse befestigten Stab verankert ist. Das obere Bandende reagiert dabei auf die relative Abwärtsbewegung des Oberschenkelteils, wenn dieses ein Gewicht trägt, um einen Bremsvorgang zwischen dem Band und der Achse auszulösen. Ferner ist ein Mittel zur Anpassung des Schleifwiderstandes vorgesehen, durch das das Oberschenkelteil gegen den oberen Teil des Bandes drückt, während die entsprechend angebrachten Öffnungen in dem Oberschenkelteil jede Abwärtsbewegung der Achse einschränken. Dieser schwenkbare Mechanismus für die bekannte Gliedmaßenprothese besteht aus folgenden Teilen:
- einem ersten Teil mit einer an einem Ende geformten Vertiefung;
- einem zweiten Teil mit einem an einem Ende geformten Schacht, der in seinem Innern eine freie Schwenkbewegung des besagten einen Endes des besagten ersten Teils zuläßt;
- einer am besagten zweiten Teil quer über dem besagten Schacht befestigten Achse;
- einem am besagten ersten Teil quer über der besagten Vertiefung und im wesentlichen parallel zur besagten Achse befestigten Stab;
- wobei die besagte Achse so angebracht ist, daß sie quer zur besagten Vertiefung verläuft;
- mindestens einem U-förmigen Bremsband, das um die besagte Achse herum innerhalb der besagten Vertiefung so angebracht ist, daß ein Ende des besagten Bandes entfernt vom besagten ersten Teil und in Berührung mit dem besagten Stab angebracht ist;
- zur Verfügung stehenden ersten Mitteln, die bewirken, daß das besagte erste Teil Kontakt mit dem anderen Ende des besagten Bremsbandes hat, um zu bewirken, daß sich dessen Enden jedesmal aufeinander zu bewegen, wenn das besagte erste Teil gegen das besagte zweite Teil gedrückt wird;
- wobei die besagten ersten Mittel bestehen aus:
- einem schwenkbar mit dem besagten Stab verbundenen Hebel, der so angebracht ist, daß er im wesentlichen parallel zum besagten Bremsband an dessen vom besagten zweiten Teil entfernten Ende verläuft;
- wobei das Ende des besagten Hebels so angebracht ist, daß es die vom besagten zweiten Teil entfernte Wand der besagten Vertiefung berührt;
- wobei der besagte Hebel zwischen seinen Enden ein Kontaktmittel aufweist, um den Kontakt mit dem besagten anderen Ende des besagten Bremsbandes herzustellen;
- wobei das besagte Kontaktmittel im wesentlichen in der gleichen Ebene liegt wie die Achsen des besagten Stabes und der besagten Achse;
- einer Gewindeschraube, die im Ende des besagten Hebels angebracht ist, um den Kontakt mit der besagten Wand herzustellen; und
- wobei das besagte erste Teil ein Paar von Löchern aufweist, die sich an den gegenüberliegenden Seiten der besagten Vertiefung befinden und es zulassen, daß die besagte Achse durch dieses Lochpaar verläuft, wodurch die besagte Achse in der Lage ist, gegen den Rand des besagten Lochpaares zu drücken, wenn die besagte Schraube angezogen wird, so daß eine Anpassung der durch das besagte Bremsband erzeugten Schleifkraft ermöglicht wird,
   oder aus
- einem ersten Teil mit einer an einem Ende geformten Vertiefung;
- einem zweiten Teil mit einem an einem Ende geformten Schacht, der in seinem Innern eine freie Schwenkbewegung des besagten einen Endes des besagten ersten Teils zuläßt;
- einer am besagten zweiten Teil quer über dem besagten Schacht befestigten Achse;
- einem am besagten ersten Teil quer über der besagten Vertiefung und im wesentlichen parallel zur besagten Achse befestigten Stab;
- wobei die besagte Achse so angebracht ist, daß sie quer zur besagten Vertiefung verläuft;
- mindestens ein U-förmiges Bremsband, das um die besagte Achse herum innerhalb der besagten Vertiefung so angebracht ist, daß ein Ende des besagten Bandes entfernt vom besagten ersten Teil und in Berührung mit dem besagten Stab angebracht ist;
- zur Verfügung stehenden ersten Mitteln, die bewirken, daß das besagte erste Teil Kontakt mit dem anderen Ende des besagten Bremsbandes hat, um zu bewirken, daß sich dessen Enden jedesmal aufeinander zu bewegen, wenn das besagte erste Teil gegen das besagte zweite Teil gedrückt wird;
- wobei die besagten ersten Mittel bestehen aus:
- einem schwenkbar mit dem besagten Stab verbundenen Hebel, der so angebracht ist, daß er im wesentlichen parallel zum besagten Bremsband an dessen vom besagte zweiten Teil entfernten Ende verläuft;
- wobei das Ende des besagten Hebels so angebracht ist, daß es die vom besagten zweiten Teil entfernte Wand der besagten Vertiefung berührt;
- wobei der besagte Hebel zwischen seinen Enden ein Kontaktmittel aufweist, um den Kontakt mit dem besagten anderen Ende des besagten Bremsbandes herzustellen;
- einem zweiten U-förmigen Bremsband, das um die besagte Achse herum angebracht ist, wobei sich der besagte Hebel zwischen den besagten Bändern befindet;
- wobei das besagte eine Ende des besagten zweiten Bandes vom besagten ersten Teil entfernt und ebenfalls in Berührung mit dem besagten Stab angebracht ist;
- wobei das besagte Kontaktmittel aus dem besagten Hebel besteht, der eine im wesentlichen parallel zum besagten Stab angebrachte Öffnung aufweist; und
- einem Dübelstab, der durch die besagte Öffnung verläuft und mit seinen Enden jeweils in Kontakt zu dem anderen Ende jedes der besagten Bremsbänder steht.

Eine ähnliche Prothese beschreibt auch die Druckschrift US-A-4 549318.

Nach der US-A-4,135,254 besteht eine Knieprothese aus einem Paar einen Abstand aufweisender, von der oberen Beinprothese abwärtsgerichteter Seitenklammern und einem zwischen den Klammern angebrachten Gehäuse. Quer durch das Gehäuse und bis zu den Klammern verlaufen ein vorderer und ein hinterer Schwenkzapfen, wobei der hintere Zapfen ein gewisses Spiel besitzt, um eine leichte Drehung um den vorderen Zapfen zuzulassen. Weiterhin ist ein Paar von Beinklammern ohne Spiel mit dem hinteren Zapfen verbunden, und das Gehäuse ist mit einer Sicherungsstifteinrichtung versehen, um die Beinklammern lösbar am Gehäuse festzustellen. Das Gehäuse weist ein Paar einander gegenüberstehender Bremsbacken auf, die auf die Beinklammern auftreffen und durch einen Keil aktiviert werden, der fest eingeklemmt wird, wenn auf das obere Beinteil hinter dem vorderen Schwenkzapfen ein Gewicht wirkt. Diese Knieprothese besteht aus einem Paar einen Abstand aufweisender seitlicher Stützglieder, die von einer Oberschenkelprothese abwärtsgerichtet sind, einem Kniemechanismusgehäuse zwischen den besagten seitlichen Stützgliedern, einem Paar einen Abstand aufweisender Beinklammern, die an ihren Unterenden an einer Unterschenkelprothese befestigt sind, einem vorderen Schwenkzapfen, der seitlich durch das besagte Gehäuse und durch die besagten seitlichen Stützglieder verläuft, einem hinteren Schwenkzapfen, der seitlich durch das besagte Gehäuse und durch die besagten Beinklammern verläuft, und Reibungsfeststellmitteln, die durch eine Schwenkbewegung um den besagten vorderen Schwenkzapfen aktiviert werden und dann die besagten Beinklammern in Bezug auf das besagte Kniemechanismusgehäuse unbeweglich arretieren.

Nach der FR-A-2 187 279 ist ein Kniegelenk mit durch Belastung des Prothesenträgers gesteuerter Bremse bekannt, die an einem an der Gelenkachse angelengten Schwinghebel sitzt, wobei das eine Kupplungsteil der Bremse durch die Gelenkachse selbst gebildet wird, während das andere Kupplungsteil aus einer die Gelenkachse umschließenden geschlitzten Achsschelle besteht, deren unterer Schenkel den an dem Gelenkoberteil über eine Schwingachse angelenkten Schwinghebel bildet, während der obere Schenkel als frei beweglicher Klemmhebel ausgebildet ist, auf dem sich das Gelenkoberteil abstützt. Damit soll ein kompaktes Prothesengelenk mit einer nach außen nicht sichtbaren und gegen äußere Einflüsse geschützten Bremskupplung geschaffen sein, das kleine Abmessungen aufweist und für kosmetisch vollverkleidbare Beinprothesen in Skelettbauweise geeignet sein soll. Die Kniegelenkachse bildet bei diesem Kniegelenk einen Teil der Bremskupplung, wodurch das Kniegelenk kleinste Abmessungen erhalten soll.

Die bekannten Kniegelenke zeigen, daß zum Teil neben aufwendigen Konstruktionen, bei denen ein hoher Verschleiß wegen der vielen, miteinander zusammenwirkenden Teilen gegeben ist, nicht gewährleistet ist, daß das Auffangen einer unkontrollierten Beugung unter vertikaler Belastung ausschließlich in der Flexionsphase wirksam wird bzw. erfolgt. Es ist auch nicht gesichert, daß dann alle Extensionsbewegungen des Kniegelenkes frei beweglich sind.

Es ist daher Aufgabe der vorliegenden Erfindung, das eingangs genannte Kniegelenk dahingehend zu verbessern, daß das Auffangen einer unkontrollierten Beugung unter vertikaler Belastung nur noch in der Flexionsbewegung wirksam wird, während alle Extensionsbewegungen des Kniegelenkes frei beweglich bleiben müssen. Hierbei soll das selbsttätige Festlaufen ohne Reibungsfriktion auf einen Bremsbelag möglich sein. Insbesondere soll das Kniegelenk einfach und verschleißmindernd aufgebaut sein.

Diese Aufgabe wird durch die Kombination der im Patentanspruch 1 aufgeführten Merkmale gelöst.

Mit einem derart erfindungsgemäß ausgebildeten Kniegelenk sind vorteilhafterweise alle Extensionsbewegungen, d.h. nach vorn gerichtete Bewegungen, eines Kniegelenkes möglich, selbst dann, wenn eine ungewollte vertikale Belastung auftritt. Der Klemmkörper und der Walzenkörper wirken wie ein Freilauf, der lediglich bei einer vertikalen Belastung die Beugebewegung sperrt und damit ein (weiteres) Einknicken verhindert. Insbesondere wird dadurch der gesamte dynamische Fortbewegungsprozess des Patienten nicht gestört, da die Vorwärtsbewegung des gesamten Prothesenkörpers sich ohne Einengung der Streckbewegung der Prothese frei auswirken kann, wenn eine Vertikalbelastung auftritt. Wenn jedoch die Streckbewegung ungehindert ablaufen kann, kann Stürzen weitgehend vorgebeugt werden. Trotz des vergleichsweise einfachen Aufbaus des Kniegelenkes bietet dieses einen minimierten Trageverschleiß.

Zur drehsicheren Befestigung des Walzenkörpers mit den Gabelarmen des Teils zur Aufnahme des Unterschenkelrohres bieten sich vorzugsweise zwei Möglichkeiten an:
Zum einen kann der Walzenkörper an seinen Stirnseiten jeweils mindestens eine, vorzugsweise drei, Gewinde-Sacklochbohrungen oder von Stirnseite zu Stirnseite durchgehende Gewindebohrungen aufweisen, die mit entsprechenden Bohrungen der Gabelarme des Teils zur Aufnahme des Unterschenkelrohres zur Deckung gebracht sind und jeweils mittels Befestigungsschrauben fest miteinander verbunden sind. Zum anderen kann der Walzenkörper stirnseitig jeweils eine oder mehrere Parallelrippen aufweisen, die durch Schlitze in den Gabelarm hindurchführbar sind. Vorzugsweise weisen die Gabelarme zusätzlich noch Führungswülste auf, über die in Verbindung mit einer in einer zentralen Gewindebohrung des Walzenkörpers eingedrehten Schraube der Walzenkörper positionierbar bzw. ausrichtbar ist.

Wegen der mechanischen Belastung, der der Walzenkörper ausgesetzt ist, besteht dieser vorzugsweise aus einem auf 60 RC vergüteten Stahlteil.

Aus dem gleichen Grund sollte der Klemmkörper eine entsprechend hohe Härte haben oder, nach einer Weiterbildung der Erfindung, im Bereich der Anlage an den Walzenkörper ein austauschbares, vorzugsweise auf 60 RC vergütetes Stahlteil aufweisen. Zur besseren Bewegungssperrung und um eine größere Bremsfläche zu haben, ist das Stahlteil im Bereich der Anlage an den Walzenkörper abgeflacht und aus verschleißmindernden Gründen mit einer gehärteten Klemmfläche ausgestattet. Die Funktionsfähigkeit des Klemmkörpers bzw. des Stahlteils kann dadurch verlängert werden, daß das Stahlteil im Klemmkörper justierbar gelagert ist. Bei fortgeschrittenem Verschleiß wird das Stahlteil - in etwa eine Zylinderwalze - weiter aus seiner Aufnahme im Klemmkörper herausgedreht, so daß die erforderliche Bremswirkung wieder eingestellt werden kann. Der Klemmkörper selbst besteht vorzugsweise aus Titan oder einer Titanlegierung.

Um ein Ausheben des Klemmkörpers während der Knieentlastung oder bei der Streckbewegung zu unterstützen, ist im Kniegelenkgehäuse eine Druckfeder angeordnet, die sich vorzugsweise stufenlos oder auf diskrete, vorgebbare Werte einstellen läßt.

Eine weitere Einstellmöglichkeit bzw. Regulierung bietet die im Gehäuse angeordnete Stellschraube, mit der das Spiel zwischen dem Walzenkörper und dem Klemmkörper justierbar ist. Auch diese Stellschraube kann vorzugsweise stufenlos oder auf diskrete Werte einstellbar sein.

Schließlich weist das Kniegelenkgehäuse noch einen Befestigungszapfen für den Streckzug im unteren Bereich des Kniegelenkgehäuses zwischen dem Walzenkörper und der Schwenkachse auf, der vorzugsweise austauschbar angeordnet ist.

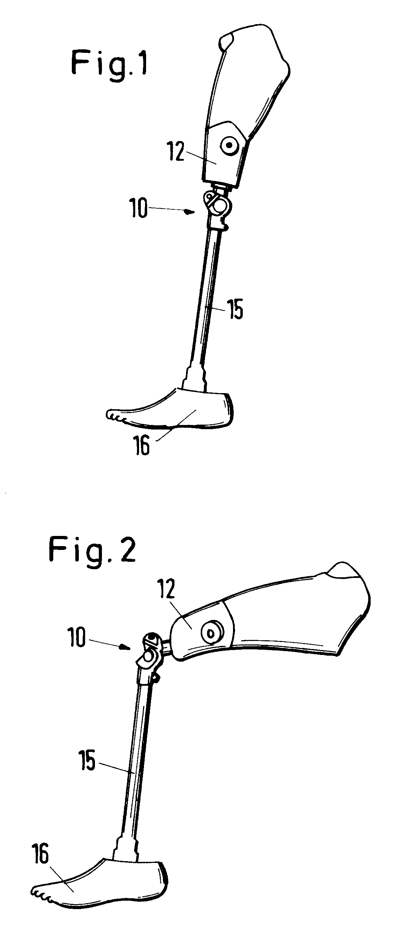

Die Erfindung wird im folgenden anhand eines in den Zeichnungen dargestellten Ausführungsbeispiels näher erläutert. Es zeigen
Fig. 1 ein mit einem Oberschenkelschaft und einem Unterschenkelrohr verbundenes Kniegelenk in gestreckter Stellung in Seitenansicht,
Fig. 2 dieselbe Anordnung in gebeugter Stellung, ebenfalls in Seitenansicht,

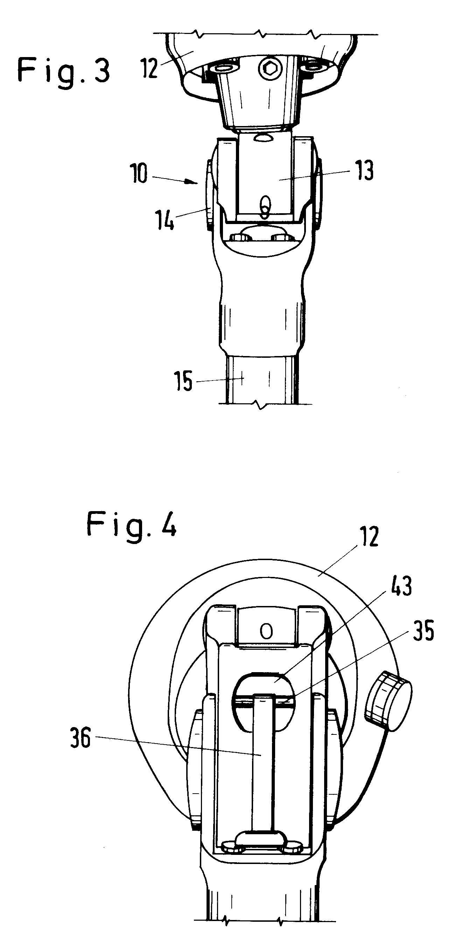

Fig. 3 eine vergrößerte Darstellung des Kniegelenkes in gestreckter Stellung in Frontansicht,
Fig. 4 die vergrößerte Ansicht nach Fig. 3 in gebeugter Stellung,
Fig. 5 eine vergrößerte Darstellung des Kniegelenkes in gebeugter Stellung in Seitenansicht,
Fig. 6 eine schematische Querschnittsdarstellung des erfindungsgemäßen Kniegelenkes,
Fig. 7 u. 8 unterschiedliche Seitenansichten des Klemmkörpers,
Fig. 9 eine auf den Walzenmantel des Walzenkörpers gerichtete Seitenansicht,
Fig. 10 denselben Walzenkörper mit Seitenansicht auf die Stirnseite,
Fig. 11 das Teil zur Aufnahme des Unterschenkelrohres in einer Draufsicht,
Fig. 12 dasselbe Teil nach Fig. 11 in einer Seitenansicht,
Fig. 13 das Kniegelenkgehäuse in einer Seitenansicht,
Fig. 14 das Kniegelenkgehäuse in einer Draufsicht von unten gesehen,
Fig. 15 dasselbe Teil nach Fig. 14 in einer Draufsicht von oben gesehen und
Fig. 16 das Kniegelenkgehäuse in einer Draufsicht von vorn betrachtet.

Das künstliche Kniegelenk 10 besitzt einen Zapfen 11 zur Befestigung an einem Oberschenkelschaft 12, wobei der Zapfen 11 Teil des Klemmkörpers 13 ist. Der Klemmkörper 13 ist in einem Kniegelenkgehäuse 14 schwenkbar befestigt, wobei das Unterschenkelrohr 15 mit endseitig befestigtem, künstlichem Fuß 16 über ein Teil 26 und einen Gabelarm 23 drehgesichert mit dem drehbaren, im Gehäuse 14 gelagerten Walzenkörper 19 verbunden ist, wobei das Kniegelenkgehäuse 14 um den im wesentlichen zylindrischen, eine horizontale Achse 18 aufweisenden Walzenkörper 19 drehbar ist.

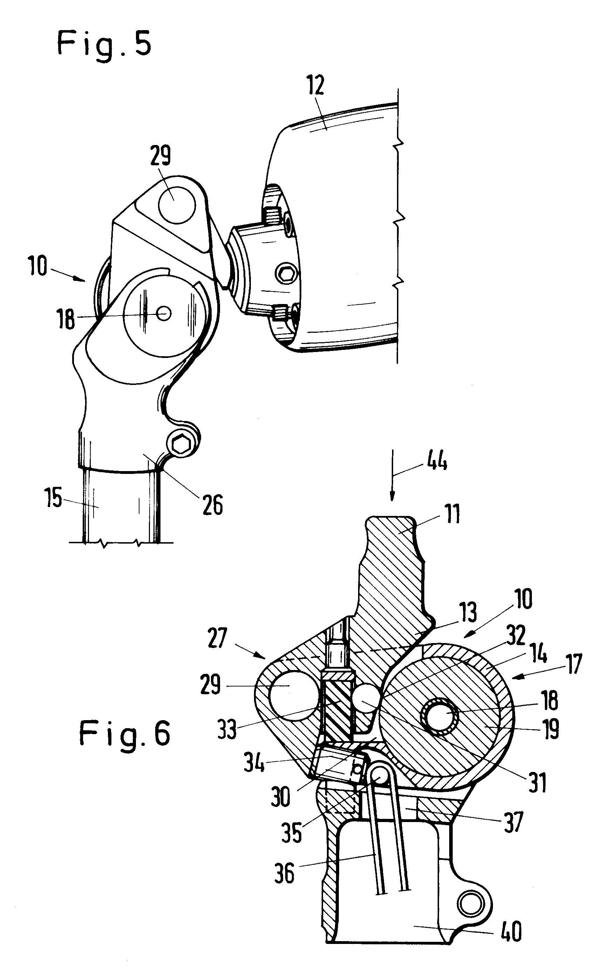

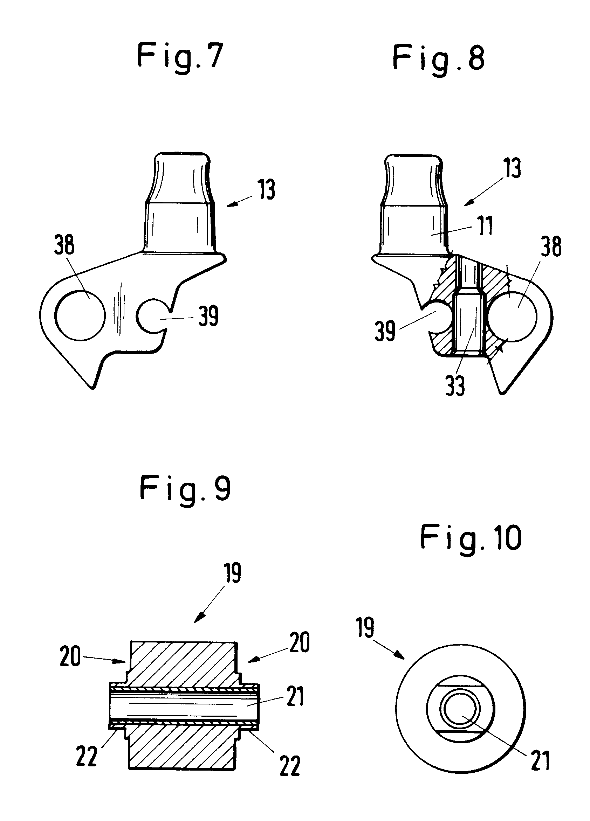

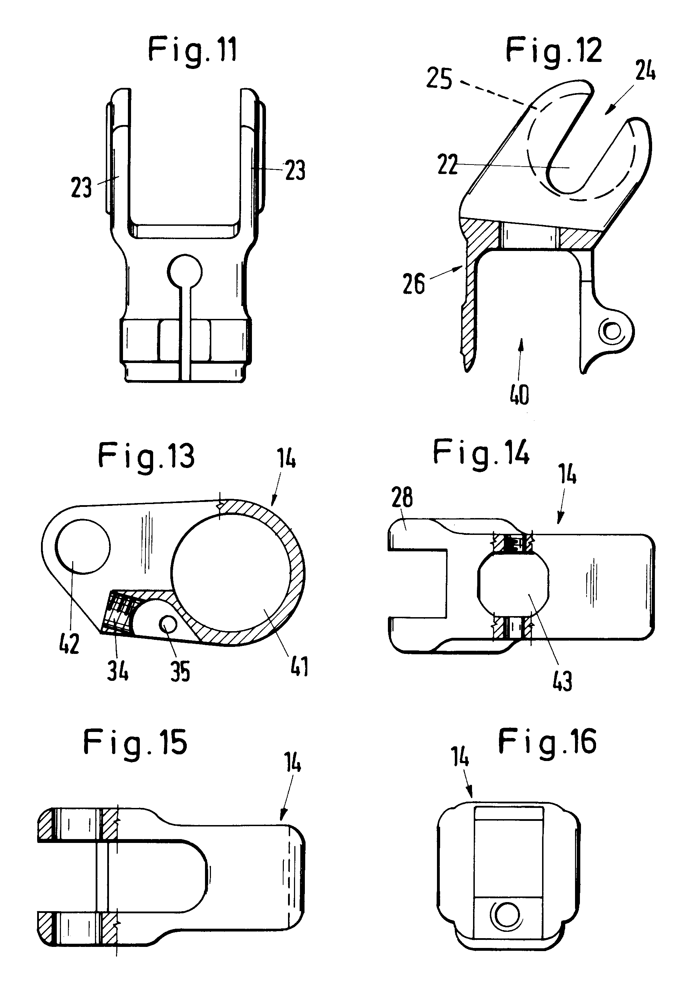

Wie insbesondere Fig. 6 entnehmbar ist, ist im hinteren Bereich 17 des Kniegelenkes 10 im Kniegelenkgehäuse 14 der Walzenkörper 19 gelagert, der im einzelnen in den Fig. 9 und 10 dargestellt ist. Die Stirnseiten 20 des Walzenkörpers 19 weisen eine (hier durchgehende) zentrale Gewindebohrung 21 zur Verschraubung des Walzenkörpers 19 mit den Gabelarmen 23 des in den Fig. 11 und 12 dargestellten Teils 26 zur Aufnahme des Unterschenkelrohres 15 auf. Wie in Fig. 12 angedeutet, besitzt jede der Stirnseiten 20 des Walzenkörpers 19 noch mindestens eine Parallelrippe 22, die in der in Fig. 12 dargestellten Art in einen Schlitz 24 jedes Gabelarmes 23 einführbar ist. Die Führungswulst 25 der Gabelarme 23 dient zur Positionierung der Gabelarme 23 gegenüber dem mit ihnen fest verbundenen Walzenkörper 19. Zur Befestigung dient eine nicht dargestellte Schraube mit möglichst breitflächigem Kopf.

Im vorderen Bereich 27 ist das Gehäuse mit einer gabelförmigen Aufnahme 28 für eine Schwenkachse 29 des Klemmkörpers 13 versehen. Die Schwenkachse 29 besteht aus einem in der gabelförmigen Aufnahme 28 gelagerten Bolzen, der eine Bohrung 38 des Klemmkörpers 13 durchgreift (Fig. 7 und 8). Im Bereich 30, in dem der Klemmkörper 13 und der Walzenkörper 19 bei vertikaler Belastung schließlich zur Anlage kommen, ist in den Klemmkörper 13 ein Stahlteil 31 in eine Öffnung 39 eingesetzt. Das Stahlteil 31 selbst besitzt eine abgeflachte und gehärtete Klemmfläche 32.

Der vordere Bereich 27 ist definiert durch die Vorderseite des Knies und der hintere Bereich 17 durch die Rückseite des Knies des menschlichen Beines (S. Figur 6). Die Druckfeder 33 (Fig. 6 und 8) hat die Aufgabe, die Ausrastspannung des Kniegelenkes zu unterstützen.

Über eine im Kniegelenkgehäuse 14 aufgenommene Stellschraube 34 kann der Klemmkörper 13 in seiner Lage zwischen dem Walzenkörper 19 und dem Stahlteil 31 stufenlos justiert werden. Der Befestigungszapfen 35 für den Streckzug 36 ist im unteren Bereich 37 des Kniegelenkgehäuses 14 horizontal liegend befestigt. Das in den Fig. 11 und 12 dargestellte Teil 26 besitzt im unteren Bereich eine Ausnehmung 40 zur Aufnahme des Unterschenkelrohres 15 mit nach dem Stand der Technik üblichen Klemmungen.

Das Kniegelenkgehäuse 14 ist in den Fig. 13 bis 16 näher dargestellt. Es besitzt insbesondere im hinteren Bereich 17 (in Fig. 13 rechts dargestellt) eine nahezu geschlossene Ausnehmung 41 mit zylinderförmigem Innenmantel zur drehbaren Lagerung des Walzenkörpers 19. Diese Ausnehmung ist lediglich an der dem Klemmkörper 13 zugewandten Seite unter Freigabe des Bereiches offen, in dem der Klemmkörper bzw. das Stahlteil 31 und der Walzenkörper 19 zur arretierenden Anlage kommen. Die gabelförmige Aufnahme 28 im hinteren Bereich 27 (in Fig. 13 links dargestellt) besitzt in beiden Gabeln Bohrungen 42, in denen ein Bolzen als Schwenkachse 29 ruht. Die Durchbrechungen 43 im unteren Bereich 37 des Gehäuses 14 dient vornehmlich zur Durchführung des Streckzuges 36, der um den Befestigungszapfen 35 greift.

Wesentliches Element des Kniegelenkes ist die absolut drehgesicherte Verbindung zwischen dem Walzenkörper 19 und dem Gabelarm 23 bzw. dem Teil 26 zur Aufnahme des Unterschenkelrohres 15 in Verbindung mit dem Klemmkörper 13 und dessen Lagerung auf der Schwenkachse 29. Bei jeder vertikalen Belastung, siehe Pfeil 44 in Fig. 6, schwenkt der Zapfen soweit nach vorn, bis er bzw. das Stahlteil 31 auf dem Walzenkörper zur Anlage kommt. Hierdurch bleiben alle Extensionsbewegungen des Kniegelenkes, d.h. ohne Beeinträchtigung der Streckbewegung der Prothese, erhalten. Ein ungewolltes Einknicken ist nicht mehr möglich.

Das Kniegelenk kann über die Stellschraube 34 hinsichtlich der Position des Klemmkörpers 13 zu dem Walzenkörper 19 ebenso justiert werden wie hinsichtlich der das Ausheben unterstützenden Kraft der Druckfeder 33.

## Patentansprüche

1. Künstliches Kniegelenk (10) mit einem einen Zapfen (11) zur Befestigung an einem Oberschenkelschaft (12) aufweisenden Klemmkörper (13), der gegen einen in einem Kniegelenkgehäuse (14) befindlichen Bremskörper (19) schwenkbar und an ihm bei vertikaler Kniegelenkbelastung die Fortführung der Schwenkbewegung sperrend anlegbar ist, und einem eine Ausnehmung zur Aufnahme des Unterschenkelrohres (15) einer Beinprothese aufweisenden Teil sowie einem das Kniegelenk in die Streckposition rücktreibenden Streckzug (36), wobei das Kniegelenkgehäuse (14) im hinteren Bereich (17) um einen im wesentlichen zylindrischen, eine horizontale Achse (18) aufweisenden Walzenkörper (19) drehbar gelagert ist, dessen Stirnseiten (20) drehgesichert mit den Gabelarmen (23) des Teils (26) zur Aufnahme des Unterschenkelrohres (15) fest verbunden sind, und im vorderen Bereich (27) eine gabelförmige Aufnahme (28) aufweist, zwischen deren seitlichen Armen eine Schwenkachse (29) gelagert ist, um die der Klemmkörper (13) derart zwischen einer Beuge- und einer Streckposition schwenkbar gelagert ist, daß der Klemmkörper (13) bei vertikaler Belastung gegen den Walzenkörper (19) zur eine Flexion (Beugebewegung) verhindernden sperrenden Anlage kommt.

2. Kniegelenk nach Anspruch 1, dadurch gekennzeichnet, daß der Walzenkörper (19) an seinen Stirnseiten (20) jeweils mindestens eine, vorzugsweise drei, Gewinde-Sacklochbohrung oder von Stirnseite zu Stirnseite (20) durchgehende Gewindebohrung aufweist, die mit entsprechenden Bohrungen der Gabelarme (23) des Teils (26) zur Aufnahme des Unterschenkelrohres (15) kongruent ist (sind), und jeweils eine Befestigungsschraube aufnimmt (aufnehmen).

3. Kniegelenk nach Anspruch 1, dadurch gekennzeichnet, daß der Walzenkörper (19) stirnseitig jeweils eine oder mehrere Parallelrippe(n) (22) aufweist, die durch Schlitze (24) in den Gabelarmen (23) hindurchführbar ist (sind).

4. Kniegelenk nach Anspruch 3, dadurch gekennzeichnet, daß die Gabelarme (23) zusätzlich Führungswülste (25) aufweisen, über die in Verbindung mit einer in einer zentralen Gewindebohrung (21) des Walzenkörpers (19) eingedrehten Schraube der Walzenkörper (19) positionierbar bzw. ausrichtbar ist.

5. Kniegelenk nach mindestens einem der Ansprüche 1 bis 4, dadurch gekennzeichnet, daß der Walzenkörper (19) ein auf 60 RC vergütetes Stahlteil ist.

6. Kniegelenk nach mindestens einem der Ansprüche 1 bis 5, dadurch gekennzeichnet, daß der Klemmkörper (13) im Bereich (30) der Anlage an den Walzenkörper (19) ein austauschbares, vorzugsweise auf 60 RC vergütetes Stahlteil (31) aufweist.

7. Kniegelenk nach Anspruch 6, dadurch gekennzeichnet daß das Stahlteil (31) im Bereich (30) der Anlage an den Walzenkörper (19) abgeflacht ist und vorzugsweise eine gehärtete Klemmfläche (32) aufweist.

8. Kniegelenk nach Anspruch 6 oder 7, dadurch gekennzeichnet, daß das Stahlteil (31) im Klemmkörper (13) justierbar gelagert ist.

9. Kniegelenk nach mindestens einem der Ansprüche 6 bis 8, dadurch gekennzeichnet, daß der Klemmkörper (13) aus Titan oder einer Titanlegierung besteht.

10. Kniegelenk nach mindestens einem der Ansprüche 1 bis 9, dadurch gekennzeichnet, daß das Kniegelenkgehäuse (14) eine Druckfeder (33) aufweist, deren wirksames Ende das Ausheben des Klemmkörpers (14) bei Entlastung oder bei der Streckbewegung unterstützt.

11. Kniegelenk nach mindestens einem der Ansprüche 1 bis 10, dadurch gekennzeichnet, daß das Kniegelenkgehäuse (14) eine Stellschraube (34) zur Einstellung bzw. Regulierung des Spiels zwischen dem Walzenkörper (19) und dem Klemmkörper (13) aufweist.

12. Kniegelenk nach Anspruch 10 oder 11, dadurch gekennzeichnet, daß die Stellschraube (34) und/oder die Druckfeder (33) stufenlos oder auf diskrete Werte einstellbar ist (sind).

13. Kniegelenk nach mindestens einem der Ansprüche 1 bis 12, dadurch gekennzeichnet, daß der Befestigungszapfen (35) für den Streckzug (36) im unteren Bereich (37) des Kniegelenkgehäuses (14) zwischen dem Walzenkörper (19) und der Schwenkachse (29) austauschbar angeordnet ist.

## Claims

1. Artificial knee joint (10) with a clamping member (13) provided with a pivot (11) for attachment to a thigh shaft (12), which is swivelable against a braking member (19) located in a knee housing (14) and, when the knee joint is subjected to a vertical load, can be made to bear upon the same so as to block the continuation of the swivel motion, and a part provided with a recess for receiving the lower leg tube (15) of a leg prosthesis as well as a traction strap (36) which urges the knee joint back into the stretched position, in which the knee joint housing (14), within the rear area (17), is rotatably supported about an essentially cylindrical roll member (19) having a horizontal axis (18), whose front ends (20) are, while secured against rotation,, rigidly connected to the bifurcate arms (23) of the part (26) which accommodates the lower leg tube (15) and which possesses, within the front area (27), a bifurcate accommodating means (28), between whose lateral arms a swivel axis is supported, about which the clamping member (13) is swivelably supported between a bent and a stretched position in such a way that the clamping member (13), when subjected to a vertical load, comes to bear upon the roll member (19) so as to impede a flexion (bending movement).

2. Knee joint according to Claim 1, characterized in that the roll member (19), on its front ends (20), is provided in each case with at least one, but preferably three, threaded blind end bores or, front end to front end (20), with a threaded through bore which is (are) congruent with corresponding bores of the bifurcate arms (23) of the part (26) intended to receive the lower leg tube (15) and receives (receive) one fastening screw each.

3. Knee joint according to Claim 1, characterized in that the roll member (19), on the front-end side, possesses in each case one or several parallel rib(s), which can be passed through slots (24) in the bifurcate arms (23).

4. Knee joint according to Claim 3, characterized in that the bifurcate arms (23) are additionally provided with guide beads (25), with the aid of which, in connection with a screw turned into a central threaded bore (21) of the roll member (19), the roll member (19) can be positioned or aligned.

5. Knee joint according to at least one of Claims 1 to 4, characterized in that the roll member (19) is a steel component tempered to 60 RC.

6. Knee joint according to at least one of Claims 1 to 5, characterized in that the clamping member (13), within the area (30) where it bears upon the roll member (19), is provided with a steel component (31) which is preferably tempered to 60 RC.

7. Knee joint according to Claim 6, characterized in that the steel component (31), within the area (30) where it bears upon the roll member (19), is flattened and preferably possesses a hardened clamping surface (32).

8. Knee joint according to Claim 6 or 7, characterized in that the steel component (31) in the clamping member (13) is supported so as to be adjustable.

9. Knee joint according to at least one of Claims 6 to 8, characterized in that the clamping member (13) is comprised of titanium or titanium alloy.

10. Knee joint according to at least one of Claims 1 to 9, characterized in that the knee joint housing (14) is provided with a compressions spring (33), the active end of which assists in the lifting out of the clamping member (14) when the load is removed or when executing a stretching movement.

11. Knee joint according to at least one of Claims 1 to 10, characterized in that the knee joint housing (14) is provided with a setscrew (34) for setting or regulating the play between the roll member (19) and the clamping member (13).

12. Knee joint according to Claim 10 or 11, characterized in that the setscrew (34) and/or the compression spring (33) is (are) adjustable so as to be infintely variable or to discrete values.

13. Knee joint according to at least one of Claims 1 to 12, characterized in that the securing peg (35) of the traction strap (36), within the lower area (37) of the knee joint housing (14), is interchangeably disposed between the roll member (19) and the swivel axis (29).

## Revendications

1. Articulation artificielle de genou (10) avec un corps de serrage (13), qui présente un tourillon (11) pour la fixation à un tronc de cuisse (12), qui est pivotant contre un corps de freinage (19) qui se trouve dans un boîtier d'articulation de genou (14), la poursuite du mouvement de pivotement pouvant être mise en appui de blocage contre lui, lors d'une sollicitation verticale de l'articulation de genou, et avec une partie qui présente un creux pour loger le tube de jambe (15) d'une prothèse de jambe ainsi qu'avec un palan d'extension (36) qui repousse l'articulation de genou dans la position d'extension, le boîtier d'articulation de genou (14) étant positionné en étant rotatif dans la zone postérieure (17) autour d'un corps de cylindre (19), substantiellement cylindrique, qui présente un axe horizontal (18), dont les faces avant (20) sont reliées de manière fixe en étant bloquées contre la rotation aux bras de fourche (23) de la partie (26) qui reçoit le tube de jambe (15), le boîtier d'articulation de genou présentant, dans la zone antérieure (27) un logement en forme de fourche (28) entre les bras latéraux duquel est positionné un axe de pivorement (29) autour duquel le corps de serrage (13) est positionné en étant pivotant entre une position de flexion et une position d'extension de telle manière que le corps de serrage (13), lors d'une sollicitation verticale, vient se placer contre le corps de cylindre (19) en un appui de blocage qui empêche une flexion (un mouvement de fléchissement).

2. Articulation de genou selon la revendication 2, **caractérisée en ce** que le corps de cylindre (19) présente, sur ses faces avant (20), respectivement une, et de préférence trois, trou(s) borgne(s) fileté(s) ou une forure filetée traversante de face avant à face avant (20) qui est (sont) congru(s) avec les forures correspondantes des bras de fourche (23) de la partie (26) qui reçoit le tube de jambe (15) et qui reçoit (reçoivent) respectivement une vis de fixation.

3. Articulation de genou selon la revendication 1, **caractérisée en ce** que le corps de cylindre (19) présente sur la face avant respectivement une ou plusieurs nervure(s) parallèle(s) (22) qui peut (peuvent) traverser des fentes (24) dans les bras de fourche (23).

4. Articulation de genou selon la revendication 3, **caractérisée en ce** que les bras de fourche (23) présentent en plus des bourrelets de guidage (25) par lesquels le corps de cylindre (19) peut être positionné ou encore aligné en relation avec une vis qui est vissée dans une forure filetée centrale (21) du corps de cylindre (19).

5. Articulation de genou selon au moins l'une des revendications 1 à 4, **caractérisée en ce** que le corps de cylindre (19) est une pièce d'acier traitée à 60 RC.

6. Articulation de genou selon au moins l'une des revendications 1 à 5, **caractérisée en ce** que le corps de serrage (13) présente, dans la zone (30) de l'appui contre le corps de cylindre (19), une pièce d'acier (31) remplaçable, de préférence traitée à 60 RC.

7. Articulation de genou selon la revendication 6, **caractérisée en ce** que la pièce d'acier (31) est aplatie dans la zone (30) de l'appui contre le corps de cylindre (19) et qu'elle présente, de préférence, une surface de serrage durcie (32).

8. Articulation de genou selon la revendication 6 ou 7, **caractérisée en ce** que la pièce d'acier (31) est positionnée dans le corps de serrage (13) en étant ajustable.

9. Articulation de genou selon au moins l'une des revendications 6 à 8, **caractérisée en ce** que le corps de serrage (13) est constitué par du titane ou un alliage au titane.

10. Articulation de genou selon au moins l'une des revendications 1 à 9, **caractérisée en ce** que le boîtier d'articulation de genou (14) présente un ressort de compression (33) dont l'extrémité de travail soutient l'enlèvement du corps de serrage (14), lors de la décharge ou lors du mouvement d'extension.

11. Articulation de genou selon au moins l'une des revendications 1 à 10, **caractérisée en ce** que le boîtier d'articulation de genou (14) présente une vis de réglage (34) pour régler ou réguler le jeu entre le corps de cylindre (19) et le corps de serrage (13).

12. Articulation de genou selon la revendication 10 ou 11, **caractérisée en ce** que la vis de réglage (34) et/ou le ressort de compression (33) est (sont) réglable(s) en continu ou à des valeurs discrètes.

13. Articulation de genou selon au moins l'une des revendications 1 à 12, **caractérisée en ce** que le tourillon de fixation (35) pour le palan d'extension (36) est placé en étant remplaçable dans la zone inférieure (37) du boîtier d'articulation de genou (14) entre le corps de cylindre (19) et l'axe de pivotement (29).
